# FASCICULE DE BREVET EUROPEEN

(11) **EP 0 728 460 B1**
(45) Date de publication et mention de la délivrance du brevet: **27.08.1997**
(21) Numéro de dépôt: 96400210.9
(22) Date de dépôt: 30.01.1996
(51) Int. Cl.: A61K 7/00

(54) **Nanoémulsion transparante à base de lipides amphiphiles non-ioniques fluides et utilisation en cosmétique ou en dermopharmacie**
Transparente Nanoemulsion auf Basis von flüssige amphiphilen nichtionischen Lipiden und ihre Verwendungen in Kosmetik und Dermapharmazie
Transparent nanoemulsion based on amphiphilic nonionic lipids and use in cosmetics or dermapharmacy

(30) Priorité: 27.02.1995 FR 9502268
(43) Date de publication de la demande: 28.08.1996
(73) Titulaire: L'OREAL, 75008 Paris (FR)
(72) Inventeur: Ribier, Alain, décédé (FR); Simonnet, Jean-Thierry, F-75011 Paris (FR); Legret, Sylvie, F-92320 Chatillon (FR)
(74) Mandataire: Lhoste, Catherine

(56) Documents cités:
- EP-A- 0 334 777
- EP-A- 0 490 053
- EP-A- 0 516 508
- EP-A- 0 572 080

## Description

La présente invention a trait à une émulsion huile-dans-eau transparente dont les globules d'huile ont une taille moyenne inférieure à 100 nm et comprenant une phase lipidique amphiphile à base de lipides amphiphiles non-ioniques liquides à une température ambiante inférieure à 45°C ainsi qu'à leur utilisation en application topique notamment en cosmétique et en dermopharmacie.

Les émulsions huile-dans-eau sont bien connues dans le domaine de la cosmétique et de la dermopharmacie notamment pour la préparation de produits cosmétiques tels que des lotions, des toniques, des sérums, des eaux de toilette.

On connaît dans l'état de la technique des microémulsions transparentes. Les microémulsions ne sont pas à proprement parler des émulsions ; ce sont des solutions transparentes de micelles, c'est-à-dire que l'huile présente y est solubilisée grâce à la présence conjointe de tensioactifs et de co-tensioactifs et grâce, généralement, à une forte proportion de ces tensioactifs et co-tensioactifs. La taille extrèmement petite des particules, cause de leur transparence, provient de cette 〈〈 solubilisation 〉〉. Les inconvénients de ces microémulsions sont justement liés à leur forte proportion en tensioactifs, conduisant à des intolérances et entraînant un toucher collant lors de l'application sur la peau. Ainsi, le document EP-A-572080 décrit des microémulsions contenant comme huile, un parfum, et un mélange de tensioactif et de co-tensioactif, la proportion d'huile et de mélange de tensioactif et co-tensioactif étant comprise entre 0,85 et 2,5.

Afin d'obtenir des compositions transparentes ayant une apparence proche de l'eau et conduisant, après application sur la peau, à un toucher se rapportant à à celui d'une crème ou d'un lait, on a déjà mis en oeuvre des nanoémulsions comprenant des globules d'huile ayant une taille moyenne inférieure à 100 nm. Ces nanoémulsions, au contraire des microémulsions, sont de vraies émulsions où les globules d'huile sont dispersés dans une phase aqueuse, les tensioactifs étant situés à l'interface huile/phase aqueuse. La transparence de ces émulsions provient de la petite taille des globules huileux, petite taille obtenue grâce à un passage à l'homogénéisateur haute pression.

On connaît dans l'état de la technique des nanoémulsions comprenant une phase lipidique amphiphile constituée de phosphoglycérides, de l'eau et de l'huile. Ces émulsions présentent l'inconvénient d'être instables au stockage aux températures traditionnelles de conservation à savoir entre 0 et 45°C. Elles conduisent à des compositions jaunes et produisent des odeurs de rance qui se développent après quelques jours de conservation. Elles sont décrites dans le brevet EP-A-406 162.

On connaît également des nanoémulsions comprenant l'association d'un alcool gras à chaîne longue et/ou d'un acide gras à chaîne longue et d'un tensio-actif du type savon d'acide gras à chaîne longue, formant un gel dont la température de transition de phase est supérieure à 60°C. Ces émulsions sont préparées à des températures élevées supérieures à 70°C qui limitent l'utilisation d'actifs thermosensibles dans de telles compositions. Elles sont décrites dans la demande EP-A-615 741.

La demanderesse a découvert, de façon inattendue, de nouvelles nanoémulsions présentant tous les avantages des nanoémulsions connues sans leurs inconvénients et pas non plus les inconvénients des microémulsions et dont les globules d'huile ont une taille moyenne inférieure à 100 nm, stables au stockage entre 0 et 45°C après au moins deux mois. Les nanoémulsions conformes à l'invention sont préparées à des températures entre 20 et 45°C et sont compatibles avec des actifs thermosensibles. Elles peuvent contenir des quantités importantes d'huile tout en conservant de bonnes propriétés de transparence. Elles peuvent notamment contenir des quantités importantes de parfum et améliorer leur rémanence. Elles favorisent également la pénétration des actifs dans les couches superficielles de la peau.

La présente invention a pour objet une émulsion huile-dans-eau ayant des globules d'huile dont la taille moyenne est inférieure à 100 nm et comprenant une phase lipidique amphiphile, telle que définie dans la revendication 1.

Les lipides non-ioniques de l'invention sont préférentiellement choisis parmi les esters ou les mélange d'esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée.

On peut citer, à titre d'exemple :
- l'isostéarate de polyéthylèneglycol de poids moléculaire 400, vendu sous la dénomination PEG 400 par la société UNICHEMA ;
- l'isostéarate de diglycéryle, vendu par la société SOLVAY ;
- le laurate de glycérol comportant 2 unités de glycérol , vendu par la société SOLVAY ;
- L'oléate de sorbitane, vendu sous la dénomination SPAN 80 par la société ICI ;
- l'isostéarate de sorbitane, vendu sous la dénomination NIKKOL SI 10R par la société NIKKO ;
- le cocoate d'α-butylglucoside ou le caprate d'α-butylglucoside commercialisés par la société ULICE.

Le rapport en poids de la quantité d'huile contenue dans l'émulsion conforme à l'invention sur la quantité de phase lipidique amphiphile varie, de préférence, de 3 à 6.

Une forme particulière d'émulsion conforme à l'invention est caractérisée par le fait que la phase lipidique amphiphile contient en plus un ou plusieurs lipides amphiphiles ioniques.

Les lipides amphiphiles ioniques, utilisés dans les nanoémulsions de l'invention, sont choisis, de préférence, dans le groupe formé par les lipides anioniques neutralisés, les lipides ioniques amphotères, les dérivés alkylsulfoniques.

Ils sont plus particulèrement choisis dans le groupe formé par :
- les sels alcalins du dicetyl- et du dimyristylphosphate ;
- les sels alcalins du cholestérol sulfate ;
- les sels alcalins du cholestérol phosphate ;
- les lipoaminoacides tels que les acylglutamates mono et disodiques ;
- les sels de sodium de l'acide phosphatidique ;
- les phospholipides ;
- les dérivés alkylsulfoniques de formule : 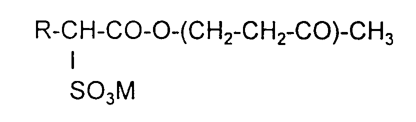 dans laquelle R représente des radicaux alkyle en C₁₆-C₂₂, en particulier les radicaux C₁₆H₃₃ et C₁₈H₃₇ pris en mélange ou séparément et M est un métal alcalin tel que le sodium.

Les lipides ioniques amphiphiles sont présents dans les nanoémulsions de l'invention, de préférence, dans des concentrations allant de 2 à 10% en poids et plus particulièrement de 5 à 10 % en poids par rapport au poids total de la phase lipidique.

Les nanoémulsions conformes à l'invention comportent une quantité d'huile allant de préférence, de 5 à 30% en poids par rapport au poids total de l'émulsion.

Les huiles pouvant être utilisées dans les émulsions de l'invention sont choisies préférentiellement dans le groupe formé par :
- les huiles animales ou végétales formées par des esters d'acide gras et de polyols, en particulier les triglycérides liquides, par exemple les huiles de tournesol, de maïs, de soja, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 19 atomes de carbone et R₁₀ représente une chaîne hydrocarbonée ramifiée contenant de 3 à 20 atomes de carbone, par exemple, l'huile de Purcellin ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- des hydrocarbures, tels que l'hexadécane et l'huile de paraffine ;
- des carbures halogénés, notamment des fluorocarbures tels que des fluoramines par exemple la perfluorotributylamine, des hydrocarbures fluorés, par exemple le perfluorodécahydronaphtalène, des fluoroesters et des fluoroethers ;
- des esters d'acide minéral et d'un alcool ;
- des éthers et des polyéthers ;
- des silicones en mélange avec au moins l'une des huiles définies ci- dessus, par exemple le décamethylcyclopentasiloxane ou le dodécaméthylcyclohexasiloxane.

Les émulsions conformes à la présente invention peuvent contenir des additifs pour améliorer la transparence de la formulation.

Ces additifs sont choisis de préférence dans le groupe formé par :
- les alcools inférieurs en C₁-C₈ tels que l'éthanol ;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.

Les additifs tels que ceux cités ci-dessus sont présents dans les émulsions de l'invention dans des concentrations allant , de préférence, de 5 à 30% en poids par rapport au poids total de l'émulsion.

Les alcools sont utilisés, de préférence, à des concentrations allant de 5 à 20% en poids.

Les glycols sont utilisés, de préférence, à des concentrations allant de 5 à 15% en poids.

En outre, l'utilisation des alcools tels que définis ci- dessus, à des concentrations supérieures ou égales à 15% en poids permet d'obtenir des émulsions sans conservateur.

Les émulsions de l'invention peuvent contenir des actifs hydrosolubles ou liposolubles, ayant une activité cosmétique ou dermopharmaceutique.Les actifs liposolubles sont dans les globules huileux de l'émulsion, tandis que les actifs hydrosolubles sont dans la phase aqueuse de l'émulsion. On peut citer, à titre d'exemples d'actif, les vitamines telles que la vitamine E et ses dérivés, les provitamines telles que le panthénol, les humectants et les filtres solaires.

Les émulsions conformes à l'invention peuvent également contenir des adjuvants utilisés pour la formulation de l'émulsion sous forme de lotion, de sérum, de crème ou de lait tels que des gélifiants, des conservateurs et des parfums.

Parmi les gélifiants utilisables, on peut citer les dérivés de cellulose tels que l'hydroxymethylpropylcellulose, les alcools gras tels que les alcools stéarylique, cétylique, béhénique, les dérivés d'algues tels que le satiagum, des gommes naturelles telles que l'adragante et des polymères synthétiques tels que les mélanges d'acides polycarboxyvinyliques commercialisés sous la dénomination CARBOPOL par la société GOODRICH et le mélange de copolymères acrylate de Na/acrylamide commercialisé sous la dénomination HOSTACERIN PN 73 par la société HOECHST.

Les globules d'huile des émulsions de l'invention, ont de préférence une taille moyenne allant de 30 à75 nm et plus préférentiellement de 40 à 60 nm. La diminution de la taille des globules permet de favoriser la pénétration des actifs dans les couches superficielles de la peau (effet véhicule).

Les émulsions selon l'invention sont incolores et éventuellement légèrement bleutées et présentent une transparence déterminée par le coefficient de transmittance mesuré à une longueur d'onde de 600 nm, allant, préférentiellement, de 30 à 90% et plus particulièrement de 50 à 80%.

Les nanoémulsions de l'invention peuvent être obtenues par un procédé, caractérisé par le fait qu'on mélange la phase aqueuse et la phase huileuse , sous agitation vive, à une température ambiante inférieure à 45°C puis qu' on effectue une homogénéisation haute pression à une pression supérieure à 10⁸ Pa et de préférence allant de 12.10⁷ à 18.10⁷ Pa. Un tel procédé permet de réaliser, à température ambiante, des nanoémulsions compatibles avec des composés actifs thermosensibles.et pouvant contenir des quantités importantes d'huiles et notamment des parfums qui renferment des corps gras, sans les dénaturer.

Un autre objet de l'invention consiste en une composition à usage topique telle qu'une composition cosmétique ou dermopharmaceutique, caractérisée par le fait qu'elle est constituée par une émulsion telle que définie précédemment.

Un autre objet de l'invention est l'utilisation des émulsions telles que définies ci-dessus comme base de produits de soin et/ou de maquillage pour la peau et/ou le visage et/ou le cuir chevelu, tels que des lotions, des sérums, des laits, des crèmes, des eaux de toilette.

Enfin, l'invention porte également sur un procédé non-thérapeutique de soin de la peau ou du cuir chevelu, caractérisé par le fait qu'on applique sur la peau ou sur le cuir chevelu une émulsion telle que définie ci-dessus.

Les exemples qui suivent, permettront de mieux comprendre l'invention, sans toutefois présenter un caractère limitatif.

Pour les exemples 1 à 7 suivants, le mode opératoire suivant est mis en oeuvre :
- dans une première phase A, on homogénéise les lipides amphiphiles avec les huiles et les actifs et adjuvants lipophiles à une température de 45°C ;
- dans une seconde phase B, on dissout les actifs et adjuvants hydrophiles à une température de 20 à 30°C ;
- puis, on mélange les phases A et B à l'aide d'un homogénéisateur à turbine puis on homogénéise à l'aide d'un homogénéisateur à haute pression du type Soavi-Niro à une pression de 1500 bars, en 7 passages en maintenant la température du produit en dessous de 35°C.
Dans le cas de l'exemple 7, on ajoute le gélifiant dans une phase C que l'on mélange aux phases A et B à l'aide d'un homogénéisateur à turbine.

### EXEMPLE 1 : Eau de soin vitaminée

| *Première phase* : | |
|---|---|
| - Isostéarate de PEG-400, vendu par la société UNICHEMA | 4,5 % |
| - Sel disodique de l'acide N-stéaroyl L-glutamique commercialisé sous la dénomination Acylglutamate HS21 par la Société AJINOMOTO (lipide amphiphile ionique) | 0,5 % |
| - Huile de jojoba | 6 % |
| - Mélange d'huiles de tournesol, hybride de tournesol, rosier muscat, pépins de cassis | 6 % |
| - Cyclométhicone | 7 % |
| - Acétate de vitamine E | 1 % |
| - Cophérol F1300 commercialisé par HENKEL | 0,2 % |
| - Palmitate de vitamine A stabilisé | 0,1 % |
| - Ethanol absolu non dénaturé | 15 % |

| *Deuxième phase:* | |
|---|---|
| - Eau déminéralisée | 54,7 % |
| - Glycérine | 5 % |

On obtient une émulsion dont la taille des globules d'huile est de 63 nm avec une transparence déterminée par le coefficient de transmittance à 600 nm, égale à 40%.

### EXEMPLE 2 : Fluide de soin

| *Première phase:* | |
|---|---|
| - Isostéarate de PEG-400, vendu par la société UNICHEMA | 4,5 % |
| - Sel disodique de l'acide N-stéaroyl L-glutamique commercialisé sous la dénomination Acylglutamate HS21 par la Société AJINOMOTO (lipide amphiphile ionique) | 0,5 % |
| - Huile de jojoba | 5 % |
| - Huile d'avocat | 5 % |
| - silicone volatile | 9 % |
| - Alcool cétylique | 1 % |
| - Acétate de vitamine E | 1 % |
| - Cophérol F1300 | 0,2 % |
| - Palmitate de vitamine A stabilisé | 0,1 % |
| - Ethanol absolu non dénaturé | 15 % |

| *Deuxième phase:* | |
|---|---|
| - Glycérol | 5 % |
| - Eau déminéralisée | qsp 100 % |

On obtient une émulsion transparente épaisse dont la taille des globules est de 53 nm et la transparence de 60%.

### EXEMPLE 3 : Fluide contour des yeux

| *Première phase:* | |
|---|---|
| - Isostéarate de PEG-400, vendu par la société UNICHEMA | 4,5 % |
| - Sel disodique de l'acide N-stéaroyl L-glutamique commercialisé sous la dénomination Acylglutamate HS21 par la Société AJINOMOTO (lipide amphiphile ionique) | 0,5 % |
| - Huile de jojoba | 5 % |
| - Huile de vaseline légère | 4 % |
| - Huile d'avocat | 4 % |
| - Silicone volatile | 6 % |
| - Acétate de vitamine E | 1 % |
| - Cophérol F1300 | 0,2 % |

| *Deuxième phase:* | |
|---|---|
| - Glycérol | 5 % |
| - Polyethylèneglycol à 8 unités d'oxyde d'éthylène | 10 % |
| - Eau déminéralisée | qsp 100 % |

On obtient une émulsion opalescente dont la taille des globules est de 65 nm et la transparence de 42%.

### EXEMPLE 4 : Fluide de soin pour le corps

| *Première phase:* | |
|---|---|
| - Isostéarate de PEG-400, vendu par la société UNICHEMA | 4,5 % |
| - Sel disodique de l'acide N-stéaroyl L-glutamique commercialisé sous la dénomination Acylglutamate HS21 par la Société AJINOMOTO (lipide amphiphile ionique) | 0,5 % |
| - Huile de vaseline légère | 7 % |
| - Huile d'avocat | 7 % |
| - Silicone volatile | 6 % |
| - Acétate de vitamine E | 1 % |
| - Ethanol absolu non dénaturé | 15 % |

| *Deuxième phase:* | |
|---|---|
| - Glycérine | 5 % |
| - Eau déminéralisée | qsp 100 % |

On obtient un fluide particulièrement fluide dont la taille des globules est de l'ordre de 50nm et la transparence de 60%.

### EXEMPLE 5 : Fluide hydratant

| *Première phase:* | |
|---|---|
| - Cocoate d'α-butylglucoside commercialisé par ULICE | 4,5 % |
| - Sel disodique de l'acide N-stéaroyl L-glutamique commercialisé sous la dénomination Acylglutamate HS21 par la Société AJINOMOTO (lipide amphiphile ionique) | 0,5 % |
| - Huile de jojoba | 5 % |
| - Huile d'avocat | 5 % |
| - Silicone volatile | 6 % |
| - Stéarylheptonate/stéarylcaprylate | 2 % |
| - Acétate de vitamine E | 1 % |
| - Ethanol absolu non dénaturé | 15 % |

| *Deuxième phase:* | |
|---|---|
| - Glycérine | 6 % |
| - Hyaluronate de sodium | 0,10 % |
| - Eau déminéralisée | qsp 100 % |

On obtient une émulsion transparente dont la taille des globules est de 52 nm et la transparence de 58%.

### EXEMPLE 6: Fluide de parfum

| *Première phase* : | |
|---|---|
| - Isostéarate de PEG-400, vendu par la société UNICHEMA | 4,5 % |
| - Sel disodique de l'acide N-stéaroyl L-glutamique commercialisé sous la dénomination Acylglutamate HS21 par la Société AJINOMOTO (lipide amphiphile ionique) | 0,5 % |
| - Huile de jojoba | 4 % |
| - Mélange d'huiles de tournesol, hybride de tornesol, rosier muscat pépins de cassis | 4 % |
| - Silicone volatile | 6 % |
| - Parfum | 6 % |
| - Ethanol absolu non dénaturé | 15 % |

| *Deuxième phase:* | |
|---|---|
| - Eau déminéralisée | 54,7 % |
| - Glycérol | 5 % |
| - Eau déminéralisée | qsp 100 % |

On obtient une lotion parfumée à forte rémanence dont la taille des globules d'huile est de 50 nm avec une transparence égale à 54%.

### EXEMPLE 7 : Baume parfumé

| *Première phase:* | |
|---|---|
| - Cocoate d'α-butylglucoside commercialisé par ULICE | 4,5 % |
| - Sel disodique de l'acide N-stéaroyl L-glutamique commercialisé sous la dénomination Acylglutamate HS21 par la Société AJINOMOTO (lipide amphiphile ionique) | 0,5 % |
| - Mélange d'huiles de tournesol, hybride de tournesol, rosier muscat pépins de cassis | 7 % |
| - Huile de vaseline légère | 7 % |
| - Silicone volatile | 6 % |
| - Parfum | 1,5 % |
| - Acétate de vitamine E | 0,5 % |
| - Ethanol absolu non dénaturé | 15 % |

| *Deuxième phase:* | |
|---|---|
| - Glycérine | 5 % |
| - Eau déminéralisée stérile | qsp 100 % |

| *Troisième phase* | |
|---|---|
| - Hydroxypropylcellulose commercialisé sous le nom METHOCEL E 4 M QG par la société DOW CHEMICAL | 0,4 % |
| - Eau déminéralisée stérile | 15 % |

On obtient un baume onctueux, non-collant, dont la taille des globules est de 54 nm et la transparence de 53%.

## Revendications

1. Emulsion huile-dans-eau transparente dont les globules d'huile ont une taille moyenne inférieure à 100 nm et comprenant une phase lipidique amphiphile, caractérisée par le fait que la phase lipidique amphiphile comprend au moins un lipide amphiphile non-ionique liquide à une température ambiante inférieure à 45°C et que le rapport en poids de la quantité d'huile sur la quantité de phase lipidique amphiphile varie de 3 à 10.

2. Emulsion selon la revendication 1, caractérisée en ce que le lipide non-ionique est un ester ou un mélange d'esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée.

3. Emulsion selon la revendication 1 ou 2 , caractérisée par le fait que le rapport en poids de la quantité d'huile sur la quantité de phase lipidique amphiphile varie de 3 à 6.

4. Emulsion selon l'une quelconque des revendications 1 à 3, caractérisée par le fait que la phase lipidique amphiphile contient en plus au moins un lipide amphiphile ionique.

5. Emulsion selon la revendication 4, caractérisée par le fait que le lipide amphiphile ionique est choisi dans le groupe formé par les lipides anioniques neutralisés, les lipides ioniques amphotères, les dérivés alkylsulfoniques.

6. Emulsion selon la revendication 4 ou 5, caractérisée par le fait que les lipides amphiphiles ioniques sont choisis dans le groupe formé par :
- les sels alcalins du dicetyl- et du dimyristylphosphate ;
- .les sels alcalins du cholestérol sulfate ;
- les sels alcalins du cholestérol phosphate ;
- les sels de lipoaminoacides ;
- les sels de sodium de l'acide phosphatidique ;
- les phospholipides ;
- les dérivés alkylsulfoniques de formule : 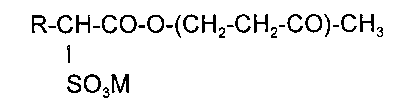 dans laquelle R représente des radicaux alkyle en C₁₆-C_{22,} pris en mélange ou séparément et M est un métal alcalin.

7. Emulsion selon l'une quelconque des revendication 4 à 6, caractérisée par le fait que le lipide amphiphile ionique est présent dans des concentrations allant de 2 à 10 % en poids par rapport au poids total de la phase lipidique.

8. Emulsion selon l'une quelconque des revendications 1 à 7, caractérisée par le fait qu'elle comprend une proportion d'huile allant de 5 à 30% en poids par rapport au poids total de l'émulsion.

9. Emulsion selon l'une quelconque des revendications 1 à 8, caractérisée par le fait que l'huile est choisie dans le groupe formé par :
- les huiles animales ou végétales formées par des esters d'acides gras et de polyols ou bien les huiles végétales ou animales de formule R₉COOR₁₀ dans laquelle R₉ représente le reste d'un acide gras supérieur comportant de 7 à 19 atomes de carbone et R₁₀ représente une chaîne hydrocarbonée ramifiée contenant de 3 à 20 atomes de carbone ;
- les huiles essentielles naturelles ou synthétiques ;
- les hydrocarbures ;
- les carbures halogénés ;
- les esters d'acide minéral et d'alcool ;
- les éthers et polyéthers ;
- les silicones en mélange avec au moins l'une des huiles définies ci-dessus.

10. Emulsion selon l'une quelconque des revendications 1 à 9, caractérisée par le fait qu'elle contient un additif permettant d'améliorer la transparence.

11. Emulsion selon la revendication 10, caractérisée par le fait que l'additif est choisi parmi les alcools inférieurs et les glycols.

12. Emulsion selon la revendication 10 ou 11, caractérisée par le fait que l'additif est présent dans des concentrations allant de 5 à 30% en poids par rapport au poids total de l'émulsion.

13. Emulsion selon la revendication 12, caractérisée par le fait que les alcools sont présents dans une concentration allant de 5 à 20% en poids par rapport au poids total de l'émulsion.

14. Emulsion selon la revendication 12, caractérisée par le fait que les glycols sont présents dans des concentrations allant de 5 à 15% en poids par rapport au poids total de l'émulsion.

15. Emulsion selon l'une quelconque des revendications 11 à 13, caractérisée par le fait qu'elle contient au moins 15% en poids d'alcool inférieur par rapport au poids total de la composition.

16. Emulsion selon l'une quelconque des revendications 1 à 15, caractérisée par le fait qu'elle contient un actif cosmétique ou dermopharmaceutique, hydrosoluble ou liposoluble.

17. Emulsion selon l'une quelconque des revendications 1 à 16, caractérisée par le fait qu'elle contient un ou plusieurs additifs choisis dans le groupe formé par les gélifiants, les conservateurs, les parfums.

18. Emulsion selon l'une quelconque des revendications 1 à 17, caractérisée par le fait que les globules d'huile ont une taille moyenne allant de 30 à 75 nm.

19. Emulsion selon l'une quelconque des revendications 1 à 18, caractérisée par le fait que les globules d'huile ont une taille moyenne allant de 40 à 60 nm.

20. Emulsion selon l'une quelconque des revendications 1 à 19, caractérisée par le fait qu'elle présente une transparence, déterminée par le coefficient de transmittance mesuré à une longueur d'onde de 600 nm, allant de 30 à 90%.

21. Composition à usage topique, caractérisée par le fait qu'elle est constituée d'une émulsion selon l'une quelconque des revendications 1 à 20.

22. Utilisation d'une émulsion telle que définie selon l'une quelconque des revendications 1 à 21 comme base de produits de soin et/ou de maquillage du corps et/ou du visage et/ou du cuir chevelu.

23. Procédé de traitement non- thérapeutique de la peau et/ou du cuir chevelu, caractérisé par le fait qu'on applique sur la peau ou le cuir chevelu une émulsion selon l'une quelconque des revendications 1 à 21.

24. Procédé de préparation d'une émulsion telle que définie selon l'une quelconque des revendications 1 à 21, caractérisé par le fait qu'on mélange la phase aqueuse et la phase huileuse, sous agitation vive, à une température ambiante inférieure à 45°C puis qu'on effectue une homogénéisation haute pression à une pression supérieure à 10⁸ Pa.

25. Procédé selon la revendication 24, caractérisé par le fait que la pression varie de 12.10⁷ à 18.10⁷ Pa.

## Claims

1. Transparent oil-in-water emulsion in which the oil globules have a mean size of less than 100 nm and which comprises an amphiphilic lipid phase, characterized in that the amphiphilic lipid phase comprises at least one non-ionic amphiphilic lipid which is liquid at an ambient temperature of less than 45°C and in that the ratio by weight of the amount of oil to the amount of amphiphilic lipid phase varies from 3 to 10.

2. Emulsion according to Claim 1, characterized in that the non-ionic lipid is an ester or a mixture of esters of at least one polyol, chosen from the group formed by polyethylene glycol containing from 1 to 60 ethylene oxide units, sorbitan, glycerol containing from 2 to 30 ethylene oxide units or polyglycerols containing from 2 to 15 glycerol units, and of at least one fatty acid containing at least one saturated or unsaturated, linear or branched, C₈-C₂₂ alkyl chain.

3. Emulsion according to Claim 1 or 2, characterized in that the ratio by weight of the amount of oil to the amount of amphiphilic lipid phase varies from 3 to 6.

4. Emulsion according to any one of Claims 1 to 3, characterized in that the amphiphilic lipid phase additionally contains at least one ionic amphiphilic lipid.

5. Emulsion according to Claim 4, characterized in that the ionic amphiphilic lipid is chosen from the group formed by neutralized anionic lipids, amphoteric ionic lipids or alkylsulphonic derivatives.

6. Emulsion according to Claim 4 or 5, characterized in that the ionic amphiphilic lipids are chosen from the group formed by:
- alkaline salts of dicetyl and dimyristyl phosphate;
- alkaline salts of cholesterol sulphate;
- alkaline salts of cholesterol phosphate;
- salts of amino acids containing fatty groups;
- sodium salts of phosphatidic acid;
- phospholipids;
- alkylsulphonic derivatives of formula: 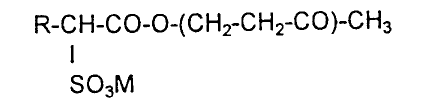 in which R
represents C₁₆-C₂₂ alkyl radicals, taken as a mixture or separately, and M is an alkali metal.

7. Emulsion according to any one of Claims 4 to 6, characterized in that the ionic amphiphilic lipid is present in concentrations ranging from 2 to 10 % by weight with respect to the total weight of the lipid phase.

8. Emulsion according to any one of Claims 1 to 7, characterized in that it comprises a proportion of oil ranging from 5 to 30 % by weight with respect to the total weight of the emulsion.

9. Emulsion according to any one of Claims 1 to 8, characterized in that the oil is chosen from the group formed by:
- animal or vegetable oils formed by esters of fatty acids and of polyols or else vegetable or animal oils of formula R₉COOR₁₀, in which R₉ represents the residue of a higher fatty acid containing from 7 to 19 carbon atoms and R₁₀ represents a branched hydrocarbon chain containing from 3 to 20 carbon atoms;
- natural or synthetic essential oils;
- hydrocarbons;
- halogenated hydrocarbons;
- esters of an inorganic acid and of an alcohol;
- ethers and polyethers;
- silicones, as a mixture with at least one of the oils defined above.

10. Emulsion according to any one of Claims 1 to 9, characterized in that it contains an additive which makes it possible to improve the transparency.

11. Emulsion according to Claim 10, characterized in that the additive is chosen from lower alcohols and glycols.

12. Emulsion according to Claim 10 or 11, characterized in that the additive is present in concentrations ranging from 5 to 30 % by weight with respect to the total weight of the emulsion.

13. Emulsion according to Claim 12, characterized in that the alcohols are present in a concentration ranging from 5 to 20 % by weight with respect to the total weight of the emulsion.

14. Emulsion according to Claim 12, characterized in that the glycols are present in concentrations ranging from 5 to 15 % by weight with respect to the total weight of the emulsion.

15. Emulsion according to any one of Claims 11 to 13, characterized in that it contains at least 15 % by weight of lower alcohol with respect to the total weight of the composition.

16. Emulsion according to any one of Claims 1 to 15, characterized in that it contains a water-soluble or liposoluble, cosmetic or dermopharmaceutical active principle.

17. Emulsion according to any one of Claims 1 to 16, characterized in that it contains one or a number of additives chosen from the group formed by gelling agents, preservatives or fragrances.

18. Emulsion according to any one of Claims 1 to 17, characterized in that the oil globules have a mean size ranging from 30 to 75 nm.

19. Emulsion according to any one of Claims 1 to 18, characterized in that the oil globules have a mean size ranging from 40 to 60 nm.

20. Emulsion according to any one of Claims 1 to 19, characterized in that it exhibits a transparency, determined by the transmittance coefficient, measured at a wavelength of 600 nm, ranging from 30 to 90 %.

21. Composition for topical use, characterized in that it is composed of an emulsion according to any one of Claims 1 to 20.

22. Use of an emulsion as defined according to any one of Claims 1 to 21 as the basis for care and/or make-up products for the body and/or for the face and/or for the scalp.

23. Process for the non-therapeutic treatment of the skin and/or of the scalp, characterized in that an emulsion according to any one of Claims 1 to 21 is applied to the skin or the scalp.

24. Process for the preparation of an emulsion as defined according to any one of Claims 1 to 21, characterized in that the aqueous phase and the oily phase are mixed, with vigorous stirring, at an ambient temperature of less than 45°C and then in that a high-pressure homogenization is carried out at a pressure greater than 10⁸ Pa.

25. Process according to Claim 24, characterized in that the pressure varies from 12 x 10⁷ to 18 x 10⁷ Pa.

## Patentansprüche

1. Durchsichtige Öl-in-Wasser-Emulsion, deren Ölkügelchen eine durchschnittliche Größe unter 100 nm aufweisen und die eine amphiphile Lipidphase umfassen, dadurch gekennzeichnet, daß die amphiphile Lipidphase mindestens ein nicht-ionisches amphiphiles Lipid umfaßt, das bei einer Umgebungstemperatur unter 45°C flüssig ist, und daß das Gewichtsverhältnis der Ölmenge zur Menge der amphiphilen Lipidphase von 3 bis 10 variiert.

2. Emulsion nach Anspruch 1, dadurch gekennzeichnet, daß es sich beim nicht-ionischen Lipid um einen Ester oder um ein Estergemisch mindestens eines Polyols, das aus der Gruppe Polyethylenglykol mit 1 bis 60 Ethylenoxideinheiten, Sorbitan, Glycerin mit 2 bis 30 Ethylenoxideinheiten und Polyglycerine mit 2 bis 15 Glycerineinheiten ausgewählt ist, und mindestens einer Fettsäure mit einer gesättigten oder ungesättigten, linearen oder verzweigten C₈-C₂₂-Alkylkette handelt.

3. Emulsion nach Anspruch 1 oder 2, dadurch gekennzeichnet, daß das Gewichtsverhältnis der Ölmenge zur Menge der amphiphilen Lipidphase von 3 bis 6 variiert.

4. Emulsion nach einem der Ansprüche 1 bis 3, dadurch gekennzeichnet, daß die amphiphlle Lipidphase zusätzlich mindestens ein ionisches amphiphiles Lipid enthält.

5. Emulsion nach Anspruch 4, dadurch gekennzeichnet, daß das ionische amphiphile Lipid aus der Gruppe neutralisierte anionische Lipide, amphotere ionische Lipide und Alkylsulfonderivate ausgewählt ist.

6. Emulsion nach Anspruch 4 oder 5, dadurch gekennzeichnet, daß die ionischen amphiphilen Lipide aus folgender Gruppe ausgewählt sind:
- Alkalisalze von Dicetyl- und Dimyristylphosphat;
- Alkalisalze von Cholesterinsulfat;
- Alkalisalze von Cholesterinphosphat;
- Salze von Lipoaminosäuren;
- Natriumsalze von Phosphatidsäure;
- Phospholipide;
- Alkylsulfonderivate der Formel: 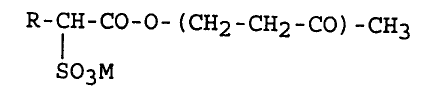 in der R C₁₆-C₂₂-Alkylreste im Gemisch oder einzeln bedeutet und M ein Alkalimetall bedeutet.

7. Emulsion nach einem der Ansprüche 4 bis 6, dadurch gekennzeichnet, daß das ionische amphiphile Lipid in Konzentrationen von 2 bis 10 Gew.-%, bezogen auf das Gesamtgewicht der Lipidphase, vorhanden ist.

8. Emulsion nach einem der Ansprüche 1 bis 7, dadurch gekennzeichnet, daß sie einen Ölanteil von 5 bis 30 Gew.-%, bezogen auf das Gesamtgewicht der Emulsion, umfaßt.

9. Emulsion nach einem der Ansprüche 1 bis 8, dadurch gekennzeichnet, daß das Öl aus folgender Gruppe ausgewählt ist:
- tierische oder pflanzliche Öle, gebildet aus Estern von Fettsäuren und Polyolen, oder pflanzliche oder tierische Öle der Formel R₉COOR₁₀, in der R₉ den Rest einer höheren Fettsäure mit 7 bis 19 Kohlenstoffatomen bedeutet, und R₁₀ eine verzweigte Kohlenwasserstoffkette mit 3 bis 20 Kohlenstoffatomen bedeutet;
- natürliche oder synthetische essentielle Öle;
- Kohlenwasserstoffe;
- Halogenkohlenstoffe;
- Ester aus anorganischen Säuren und Alkoholen;
- Ether und Polyether; und
- Silicone im Gemisch mit mindestens einem der vorstehend definierten Öle.

10. Emulsion nach einem der Ansprüche 1 bis 9, dadurch gekennzeichnet, daß sie ein Additiv enthält, das eine Verbesserung der Durchsichtigkeit ermöglicht.

11. Emulsion nach Anspruch 10, dadurch gekennzeichnet, daß das Additiv unter niederen Alkoholen und Glykolen ausgewählt ist.

12. Emulsion nach Anspruch 10 oder 11, dadurch gekennzeichnet, daß das Additiv in Konzentrationen von 5 bis 30 Gew.-%, bezogen auf das Gesamtgewicht der Emulsion, vorhanden ist.

13. Emulsion nach Anspruch 12, dadurch gekennzeichnet, daß die Alkohole in einer Konzentration von 5 bis 20 Gew.-%, bezogen auf das Gesamtgewicht der Emulsion, vorhanden sind.

14. Emulsion nach Anspruch 12, dadurch gekennzeichnet, daß die Glykole in Konzentrationen von 5 bis 15 Gew.-%, bezogen auf das Gesamtgewicht der Emulsion, vorhanden sind.

15. Emulsion nach einem der Ansprüche 11 bis 13, dadurch gekennzeichnet, daß sie mindestens 15 Gew.-% niederen Alkohol, bezogen auf das Gesamtgewicht der Zusammensetzung, enthält.

16. Emulsion nach einem der Ansprüche 1 bis 15, dadurch gekennzeichnet, daß sie einen kosmetischen oder dermopharmazeutischen, wasserlöslichen oder fettlöslichen Wirkstoff enthält.

17. Emulsion nach einem der Ansprüche 1 bis 16, dadurch gekennzeichnet, daß sie einen oder-mehrere Additive enthält, die aus der Gruppe gelbildende Mittel, Konservierungsmittel und Parfums ausgewählt sind.

18. Emulsion nach einem der Ansprüche 1 bis 17, dadurch gekennzeichnet, daß die Ölkügelchen eine durchschnittliche Größe von 30 bis 75 nm aufweisen.

19. Emulsion nach einem der Ansprüche 1 bis 18, dadurch gekennzeichnet, daß die Ölkügelchen eine durchschnittliche Größe von 40 bis 60 nm aufweisen.

20. Emulsion nach einem der Ansprüche 1 bis 19, dadurch gekennzeichnet, daß sie eine Durchsichtigkeit, bestimmt durch den bei einer Wellenlänge von 600 nm gemessenen Durchlässigkeitskoeffizienten, von 30 bis 90% aufweist.

21. Zusammensetzung zur topischen Anwendung, dadurch gekennzeichnet, daß sie aus einer Emulsion nach einem der Ansprüche 1 bis 20 besteht.

22. Verwendung der Emulsion nach einem der Ansprüche 1 bis 21 als Grundlage für Pflege- und/oder Schminkprodukte für den Körper und/oder das Gesicht und/oder die Kopfhaut.

23. Verfahren zur nicht-therapeutischen Behandlung der Haut und/oder der Kopfhaut, dadurch gekennzeichnet, daß man auf die Haut oder auf die Kopfhaut eine Emulsion nach einem der Ansprüche 1 bis 21 aufbringt.

24. Verfahren zur Herstellung einer Emulsion gemäß der Definition in einem der Ansprüche 1 bis 21, dadurch gekennzeichnet, daß man die wäßrige Phase und die Ölphase unter heftigem Bewegen bei einer Umgebungstemperatur unter 45°C mischt und anschließend eine Hochdruckhomogenisation bei einem Druck über 10⁸ Pa durchführt.

25. Verfahren nach Anspruch 24, dadurch gekennzeichnet, daß der Druck von 12 x 10⁷ bis 18 x 10⁷ Pa variiert.
